# FASCICULE DE BREVET EUROPEEN

(11) **EP 2 303 819 B1**
(45) Date de publication et mention de la délivrance du brevet: **07.05.2014**
(21) Numéro de dépôt: 09772707.7
(22) Date de dépôt: 08.06.2009
(51) Int. Cl.: C07C 17/389, C07C 21/18

(54) **PROCEDE DE PURIFICATION DE 2,3,3,3-TETRAFLUORO-1-PROPENE (HF01234yf)**
VERFAHREN ZUR AUFREINIGUNG VON 2,3,3,3-TETRAFLUOR-1-PROPEN (HFO-1234YF)
PROCESS FOR THE PURIFICATION OF 2,3,3,3-TETRAFLUORO-1-PROPENE (HFO-1234YF)

(30) Priorité: 03.07.2008 FR 0854514
(43) Date de publication de la demande: 06.04.2011
(73) Titulaire: Arkema France, 92700 Colombes (FR)
(72) Inventeur: DEVIC, Michel, 69110 Sainte Foy Les Lyon (FR); PIGAMO, Anne, 69340 Francheville (FR); WENDLINGER, Laurent, 69510 Soucieu en Jarrest (FR)
(74) Mandataire: Dang, Doris
(86) Numéro de dépôt international: PCT/FR2009/051074
(87) Numéro de publication internationale: WO 2010/001025

(56) Documents cités:
- EP-A- 0 511 612
- WO-A-01/83411
- WO-A-2008/001844
- WO-A2-2007/079431
- US-A- 3 215 747
- US-B2- 7 084 315

## Description

### DOMAINE DE L'INVENTION

L'invention a pour objet un procédé de purification du 2,3,3,3-tétrafluoro-1-propène (HFO 1234yf).

### ARRIERE-PLAN TECHNOLOGIQUE

Les hydrofluorocarbones (HFC) et en particulier les hydrofluorooléfines (HFOs), telles que le 2,3,3,3-tétrafluoro-1-propène (HFO 1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. A la différence des CFC et des HCFC, qui sont potentiellement dangereux pour la couche d'ozone, les HFOs ne contiennent pas de chlore et donc ne posent pas de problème pour la couche d'ozone.

Le HFO 1234yf peut être obtenu par déshydrofluoration d'un pentafluoropropane. Il peut s'agir du 1,1,1,2,3-pentafluoropropane (HFC 245eb) comme cela est décrit dans le document WO2008/002500 ou du 1,1,1,2,2-pentafluoropropane (HFC 245cb) comme cela est décrit dans le document WO2007/079435. Ces pentafluoropropanes sont préparés par fluoration en phase liquide ou gazeuse de composés chlorés ou chlorofluorés ou par des réactions successives de déhydrohalogénation et d'hydrogénation. Le document US-P-5396000 décrit par exemple la préparation de 1,1,1,2,3-pentafluoropropane (HFC 245eb) par déhydrohalogénation catalytique de 1,1,1,2,3,3-hexafluoropropane (HFC 236ea) en 1,2,3,3,3-pentafluoropropène-1 (HFO 1225ye), suivi d'une hydrogénation pour produire le composé recherché.

Plus généralement, le HFO 1234yf peut être obtenu par déhydrohalogénation d'un tétrafluorohalogénopropane. Il peut s'agir du 1,1,1,2-tétrafluo-3-iodopropane comme cela est décrit dans le document WO2005/108334 ou du 1,1,1,2-tétrafluo-2-chloropropane (HCFC 244bb) comme cela est décrit dans les documents WO2007/079431 et WO2008/040969.

Tous ces procédés partent de ou passent intermédiairement par des composés halogénofluorés saturés. En raison de différences de volatilité élevées entre ces composés halogénofluorés saturés et le HFO 1234yf, ceux ci peuvent généralement être séparés par des procédés classiques de distillation. Néanmoins, dans un souci d'obtention d'un produit présentant une pureté maximale, il convient d'abaisser la concentration résiduelle en impuretés saturées.

L'utilisation de solides adsorbants pour la purification de composés fluorés est déjà connue. Les traitements de purification sont usuellement effectués à la température ambiante ou au voisinage de celle-ci.

Le document JP 2002/226411 décrit la purification de 1,1,1,3,3-pentafluoropropane (HFC 245fa) comprenant entre 5 ppm et 2 % en poids d'halopropène, comme le fluoropropène et chlorofluoropropène, à l'aide d'un adsorbant solide, notamment le charbon actif.

Le document US7084315 décrit l'élimination d'impuretés oléfiniques dans un hydrofluoroalcane, typiquement l'élimination de 1-chloro-2,2-difluoroethylène (F1122) dans le 1,1,1,2-tetrafluoroéthane (F134a) sur des tamis moléculaires. Le HFO 1234yf peut être présent en tant qu'impureté.

Le document US7041264 décrit un procédé de purification d'octafluoropropane comprenant une étape de mise en contact à température élevée de l'octafluoropropane brut contenant des impuretés avec un agent de décomposition d'impuretés suivie d'une étape d'élimination de ces impuretés, par exemple à l'aide d'un adsorbant. L'agent de décomposition préféré comprend un oxyde de fer et un composé alcalino-terreux.

Le document US7094935 décrit une méthode de purification d'octafluoropropane ou d'octafluorocyclobutane à l'aide d'un adsorbant obtenu selon un procédé comprenant (i) une étape de lavage acide de charbon, suivi d'un lavage àl'eau, (ii) une étape de déoxydation et/ou déhydratation du charbon, (iii) une étape de recarbonisation entre 500-700°C et une étape d'activation à température comprise entre 700 et 900°C sous un flux gazeux comprenant un gaz inerte, du dioxyde de carbone et de la vapeur d'eau

Le document WO2007/144632 décrit l'emploi de tamis moléculaire, avec une ouverture de pores de dimensions comprises entre 3 et 5 Å pour contrôler le niveau d'humidité d'un fluide réfrigérant comprenant un fluoropropène, notamment le HFO 1234yf, éventuellement en mélange avec le iodotrifluoro méthane et/ou un lubrifiant.

Le document JP1040507, enseigne l'emploi de tamis moléculaires pour réduire la teneur de perfluorobutyne-2, impureté présente dans l'hexafluoropropène, à moins de 5 ppm.

Dans le brevet WO08001844, de l'hexafluoropropène brut est mis en contact avec un adsorbant comprenant une zéolite dont les micropores ont un diamètre moyen compris entre 3,4 et 11 Å et/ou un asdorbant a base de charbon dont les micropores ont un diamètre moyen compris entre 3,5 et 11 Å pour réduire la teneur en composés chlorés et/ou en hydrocarbures dans l'hexafluoropropène.

### DESCRIPTION DETAILLEE DE L'INVENTION

Il existe un besoin d'un procédé d'obtention du HFO 1234yf de haute pureté. L'invention a donc pour objet un procédé de purification du 1234yf à partir d'un HFO 1234yf brut comprenant des impuretés à base de composés carbonés halogénés. Les composés carbonés halogénés peuvent être insaturés ou saturés. Comme composés halogénés saturés, on peut citer notamment les HFC 245 ( pentafluoropropane), HFC 236 (hexafluoropropane), HFC 254 (tetrafluoropropane) et HCFC 244 (monochlorotetrafluoropropène).

Il a été trouvé que les impuretés à base de composés halogénés présents dans un HFO 1234yf peuvent être éliminées (de manière partielle ou totale) par la mise en contact d'un HFO 1234yf brut avec un adsorbant. Le procédé de purification, selon la présente invention, est caractérisé en ce que le HFO 1234yf, comprenant des impuretés à base de composés halogénés est mis en contact avec des tamis moléculaires ayant une ouverture de pores de diamètre moyen compris entre 5 et 9 Å.

Les tamis moléculaires, encore appelés zéolithes synthétiques, sont des composés chimiques largement utilisés dans l'industrie comme agents adsorbants, notamment pour sécher des gaz ou des liquides. Ce sont des alumino-silicates métalliques qui possèdent une structure cristalline tridimensionelle constituée par un assemblage de tétraèdres. Ces tétraèdres sont formés par quatre atomes d'oxygène qui occupent les sommets, et qui entourent soit un atome de silicium, soit un atome d'aluminium placé au centre. Ces édifices contiennent généralement des cations pour rendre le système électriquement neutre, tels ceux dérivés du sodium, du potassium ou du calcium.

Les tamis moléculaires qui conviennent sont de préférence ceux du type A et du type X et avantageusement ceux du type X.

Dans le cas des tamis moléculaires, dits du type A, les tétraèdres sont assemblés de telle manière qu'ils composent un octaèdre tronqué. Ces octaèdres sont eux-mêmes arrangés selon une structure cristalline cubique simple, formant un réseau dont les cavités ont un diamètre approximatif de 11,5 Å. Ces cavités sont accessibles par des ouvertures, ou pores, qui peuvent être partiellement bloquées au moyen de cations. Lorsque ces cations sont dérivés du sodium, ces cavités ont un diamètre d'ouverture de 4,1 Å, et l'on a alors un tamis moléculaire dit 4 A. La structure cristalline d'un tel tamis peut être représentée par la formule chimique suivante:

Na₁₂ [(AlO₂)₁₂(SiO₂)₁₂]. X H₂O

dans laquelle X qui représente le nombre de molécules d'eau appartenant à la structure (eau de cristallisation) peut atteindre 27, ce qui représente 28,5 % en poids de la zéolithe anhydre.

Après élimination de l'eau de cristallisation par chauffage à une température de l'ordre de 500 à 700°C, les cavités de ces substances sont disponibles pour l'adsorption sélective de différents gaz ou liquides. Ainsi, les pores des divers types de zéolithe ne permettent de laisser passer et de s'adsorber dans les cavités correspondantes que les molécules dont le diamètre effectif est inférieur ou égal au diamètre effectif des pores. Dans le cas du séchage de gaz ou de liquides, ce sont donc les molécules d'eau qui sont retenues par adsorption sélective à l'intérieur des cavités mentionnées précédemment, la substance à sécher n'étant quant à elle pas ou peu adsorbée.

La taille des ouvertures (ou des pores) peut du reste être modifiée selon les différents types de tamis moléculaire. Ainsi, par échange d'une grande partie des ions sodium d'un tamis moléculaire 4A par des ions potassium, on obtient le tamis moléculaire 3A, dont les pores ont un diamètre d'environ 3 Å. Le tamis moléculaire 5A est réalisé en remplaçant les ions sodium par des ions calcium, le diamètre effectif des pores étant alors de l'ordre de 5 Å.

La cellule élémentaire de la zéolithe X est un tétraèdre dont les sommets sont occupés par des polyèdres de même type que ceux présents dans la zéolithe A, chacun étant connecté à quatre autres polyèdres grâce à une sous-structure octaédrique, formée par un double-cycle contenant huit atomes d'oxygène. Le centre de chaque arête est toujours occupé par un atome d'oxygène, tandis que les atomes de silicium et d'aluminium occupent les différents sommets des polyèdres. La formule brute est de structure Na₈₈Al₈₈Si₁₀₄O₃₈₄,220H₂O.

Le procédé selon l'invention convient à la purification d'un HFO 1234yf brut ayant une pureté d'au moins 85% en poids, de préférence supérieure à 90% en poids et avantageusement supérieure à 95% en poids.

Le HFO 1234yf brut soumis à l'étape de purification peut provenir directement de l'effluent issu de l'étape de fabrication, après éventuelle séparation telle que décantation ou distillation.

Les impuretés à base de composés halogénés saturés présents dans le HFO 1234yf sont notamment le 245eb (CF₃-CHF-CH₂F), le 245cb (CF₃-CF₂-CH₃), le 236ea (CF₃-CHF-CHF₂), le 1,1,1,2 -tetrafluoro- 3 chloro propane et le tetrafluoropropane. Les impuretés à base de composés halogénés insaturés, sont notamment les fluoropropènes, tels que les 1,1,1,2,3 pentafluoropropène, le 1,1,1,3,3 pentafluoropropène et le 1,1,1 trifluoropropène.

La mise en contact avec l'adsorbant pour purifier le HFO 1234yf brut peut être effectuée en phase gazeuse ou en phase liquide à une température comprise entre -20°C et + 80°C, de préférence entre +10°C et +40°C, et sous une pression de 100 à 2200 kPa, de préférence à pression atmosphérique.

Pour le traitement en phase gazeuse, on peut utiliser un débit correspondant à une vitesse comprise entre 10 et 40 g/h de HFO 1234yf brut pour une quantité d'adsorbant comprise entre 10 et 50 g. L'exemple suivant illustre l'invention sans la limiter.

### EXEMPLE

### Exemple 1

Dans un tube en acier inoxydable de 70 cm de hauteur et de 16 mm de diamètre intérieur comportant une grille métallique dans la partie inférieure, on a placé une charge de 30 g de tamis moléculaire CECA G5 (silicoaluminate de sodium, zéolithe de type X) ayant des diamètres de pores de 7,8 Å et un volume poreux de 0,24 cm³/g. Un courant gazeux de 1234yf contenant 0,38% de 236ea, 6,42% de 245eb et 1,17% de 254 a parcouru le lit de zéolithe à température ambiante et pression atmosphérique avec un débit de 32g/h. Une quantité totale de produit de 95,6g a été introduite sous forme gazeuse à travers le lit de tamis moléculaire pendant une durée d'environ 3 heures. 88,6 g de produit ont été récupérés en sortie. Le rendement est donc de 92,7%. La composition en entrée et en sortie est donnée dans le tableau 1.

Les analyses sont effectuées avant et après passage sur le tamis moléculaire à l'aide d'un chromatographe en phase gaz équipé d'une colonne CarbopackB/1%SP1000.

**Tableau 1- résultats analytiques avant et après passage sur lit de zéolithe**

| | | | | | | | | |
|---|---|---|---|---|---|---|---|---|
| Mol% | 1234yf | 1225zc | 1225yeZ | F254 | 1225yeE | 1234zeZ | 245eb | 236ea |
| Analyse départ | 87,5 | 0,46 | 3,51 | 1,17 | 0,14 | 0,13 | 6,42 | 0,38 |
| Exemple 1 | 96,2 | 0,42 | 2,97 | <10ppm | 0,1 | <10ppm | <10ppm | <10ppm |

## Revendications

1. Procédé de purification du 2,3,3,3 tetrafluoropropène **caractérisé en ce que** le 2,3,3,3 tetrafluoropropène, comprenant des impuretés à base de composés halogénés est mis en contact avec des tamis moléculaires, ayant une ouverture de pores de diamètre moyen compris entre 5 et 9 Å.

2. Procédé de purification selon la revendication 1 **caractérisé en ce que** les tamis moléculaires sont de type X ou A.

3. Procédé de purification selon l'une quelconque des revendications précédentes **caractérisé en ce que** les impuretés à base de composés halogénés sont le 1,1,1,2,3 pentafluoropropane, le 1,1,1,2,2 pentafluoropropane et/ou le 1,1,1,2,3,3 hexafluoropropane.

## Patentansprüche

1. Verfahren zur Reinigung von 2,3,3,3-Tetrafluorpropen, **dadurch gekennzeichnet, dass** man 2,3,3,3-Tetrafluorpropen, das Verunreinigungen auf Basis halogenierter Verbindungen enthält, mit Molsieb mit einer Porenöffnung mit einem mittleren Durchmesser zwischen 5 und 9 Å in Berührung bringt.

2. Reinigungsverfahren nach Anspruch 1, **dadurch gekennzeichnet, dass** das Molsieb vom X- oder A-Typ ist.

3. Reinigungsverfahren nach einem der vorhergehenden Ansprüche, **dadurch gekennzeichnet, dass** es sich bei den Verunreinigungen auf Basis halogenierter Verbindungen um 1,1,1,2,3-Pentafluorpropan, 1,1,1,2,2-Pentafluorpropan und/oder 1,1,1,2,3,3-Hexafluorpropan handelt.

## Claims

1. Process for the purification of 2,3,3,3-tetra-fluoropropene, **characterized in that** the 2,3,3,3-tetra-fluoropropene, comprising impurities based on halogenated compounds, is brought into contact with molecular sieves, having a pore opening with a mean diameter of between 5 and 9 Å.

2. Purification process according to Claim 1, **characterized in that** the molecular sieves are of type X or A.

3. Purification process according to either one of the preceding claims, **characterized in that** the impurities based on halogenated compounds are 1,1,1,2,3-pentafluoropropane, 1,1,1,2,2-pentafluoropropane and/or 1,1,1,2,3,3-hexafluoropropane.
